Europäisches Patentamt

European Patent Office

Office européen des brevets

(19)

(11) Publication number: **0 170 760**
A1

## (12) EUROPEAN PATENT APPLICATION

(21) Application number: **84850238.1**

(22) Date of filing: **03.08.84**

(51) Int. Cl.⁴: **G 01 N 33/569**, G 01 N 33/573, C 12 Q 1/34, G 01 N 35/00, G 01 N 21/25

(43) Date of publication of application: **12.02.86**
**Bulletin 86/7**

(84) Designated Contracting States: **CH DE FR GB IT LI**

(71) Applicant: **STATENS BAKTERIOLOGISKA LABORATORIUM, S-10521 Stockholm (SE)**

(72) Inventor: **Söderholm, Jan, Luntmakargatan 71, S-11351 Stockholm (SE)**

(74) Representative: **Rosenquist, Holger et al, H Albihns Patentbyra AB Box 7664, S-103 94 Stockholm (SE)**

(54) **Serologic diagnostics of beta-streptococcal infections with the use of automatic reading with photometer for microtitration plates.**

(57) After infection with β-haemolyzing streptococci, antibodies in the patient serum is detected by neutralizing the serum with streptococcal DNase B on microtitration plates. The reaction is read off either by coloring the substrate with methyl green, illuminating it with monochromatic light and reading it by means of a photometer provided with a 640 nm interference filter, or the reading is performed by means of UV-light having a wavelength of 260 or 254 nm. Colorless DNA is used as a substrate, and the microtitration plates are made of UV-transparent plastics. By connecting a microcomputer to the photometer device, automatically processed analysis results can be obtained. Readings can be performed on one sample from a series of dilutions, or on one and the same sample at specific time intervals.

ACTORUM AG

SEROLOGIC DIAGNOSTICS OF $\beta$-STREPTOCOCCAL INFECTIONS WITH THE
USE OF AUTOMATIC READING WITH PHOTOMETER FOR MICROTITRATION
PLATES

The present invention relates to a method of serologically diagnosing $\beta$-streptococcal infections by neutralization of streptococcal DNase B with patient serum, and by using a photometer for automatic reading of the reaction on microtitration plates.

In certain infections there are obtained such low concentrations of bacteria that they can be difficult to isolate and determine by means of conventional cultivation techniques. By a direct determination of bacterial metabolic products a recognition can be possible in such cases, or a determination of the subsequent increase of titer in the antibodies directed against these metabolic products can confirm this fact. Streptococci group A form a plurality of such extracellular metabolic products of enzyme character which are partly immogeneously active.

Certain pathogenic streptococci can give rise to serious complications such as rheumatic fever and chronic nephritis. Particularly intricate from a diagnostics point of view are so-called $\beta$-haemolytic streptococci since the detection of bacteria by means of general cultivation can solely take place in the acute phase of the course of disease, whereas at a later stage there is no other choice than trying to demonstrate the presence of antibodies in the patient serum with the aid of serologic diagnostics. Antibodies against a number of different extracellular and somatic antigens can be detected in the patient's serum subsequent to an infection by $\beta$-haemolyzing streptococci. In routine diagnostics, detection of antibodies against the extracellular antigens has had its primary significance.

Beyond comparison, the antistreptolysine reaction has been the most frequently used reaction and is still considered to be superior in connection with routine diagnostics. The antistreptolysine reaction, the AS, has good reproducibility, the antigen is produced by most of the group A streptococcal strains, and the AS reaction is the one from which the longest experience is gained. There is an appreciable disadvantage in this respect, however, in that other substrates are present in serum in addition to antibodies which are capable of neutralizing the haemolytic activity in reduced streptolysine 0. In recent years the antideoxyribonuclease B reaction, the ADNase reaction, has therefore been taken up as a complement to the AS reaction due to the fact that it becomes positive in streptococcal skin infections more often than does the AS reaction. The method for the ADNase determination was worked out in 1949 already but did not attract much attention until 1958 after it was found that the $\beta$-streptococci produced four deoxyribonucleases; A, B, C and D having different characteristics. DNase B was detected almost exclusively among the group A streptococci and was most frequently and abundantly present among these streptococci.

ADNase tests are usually carried out in the form of neutralization reactions with streptococcal DNase as antigen. The principle of the test is the fact that the colored substrate will be decolorized upon addition of enzyme. Patient sera contain varying concentrations of antibodies against DNase B after streptococcal infections. The content of antibodies in serum is measured by the addition of a specific amount of serum to the enzyme prior to adding the enzyme to the substrate. The enzyme activity will be inhibited in the presence of antibodies, which will result in a reduced decomposition/decolorization of the substrate.

In its present form the test is carried out so that a constant amount of enzyme, divided into a number of equal volumes, is subjected to a series dilution of serum by 1:50, 1:100, 1:200 etc. When compared to known check sera it is possible to visually determine the degree of dilution of a patient serum at which decolorization of the substrate is inhibited in order to obtain in this manner an index on the concentration of antibodies. The reaction is carried out on so-called microtitration plates of transparent plastics measuring about 8 x 12 x 1 cm and having 12 wells in rows of 8, i.e. totally 96 wells each containing approx. 200 µl of liquid. The dispensation of the reagents is facilitated by manual and automatic equipment such as multichannel micro-pipettes etc., and thanks to the microtechniques, the consumption of reactants will be insignificant.

However, the test can also be carried out by diluting the enzyme with serum while following the decolorization of the substrate continually against time; so-called kinetic analysis.

The volume of testings has been studied in a few countries and has been found to lie within the range of approx. 4 million tests per year.

The present invention relates to a method of simplifying the evaluation of the test results by utilizing a photometer device for automatic reading of the mictrotitration plates.

Devices for automatic reading of mictrotitration plates have been developed in other fields and have been found useful in a plurality of microbiological laboratories. Examples of such apparatuses are Flow Laboratories Multiscan, Multiskan MC, Dynatech MR580, MR600, Microelisa Auto Reader, multichannel filter photometers for digital reading of alterations in the absorbance in small volumes of liquid dispensed on microtitration plates.

The decomposition of DNA with DNase can be followed visually, using DNA methyl green as a substrate. Photometric determinations are then suitably made at approx. 640 nm, but the DNA activity can also be followed within the UV-range at approx. 260 nm.

By connecting a minicomputer such as PET or ABC 800, the analysis results can then be obtained in processed form.

The performance of a test will be described in more detail in the following exemplary embodiment.

Example, the ADNase B test

The ADNase B test was carried out mainly in accordance with the method presented by Nelson, J. and associates in J. Lab. Clin. Med. 71:867, 1968.

A serum with the initial dilution of 1/25 was diluted in a dilution series of 1/2, 1/4 etc. instead of, as recommended by Nelson, in three series with the initial dilutions of 1/50, 1/60 and 1/80.

Streptococcal DNase B enzyme: DNase B enzyme from streptococci was isolated and was purified by ion exhcange chromatography, isoelectric focusing etc.

The enzyme in solution was stored undiluted at $-25^{\circ}C$ or dryfrozen at $+5^{\circ}C$, and was diluted/dissolved in a suitable pH buffer prior to use.

DNA methyl green substrate: High-polymerized calf thymus DNA was dissolved to a concentration of 0.01 (w/v) together with 0.005% - 0.01% (W/v) methyl green, purified by chloroform extraction, in e.g. 50 mM tris-HCl pH 7.8 with 10 mM $MgSO_4$ or 50 mM imidazole buffer pH 7.7 with 10 mM $MgSO_4$. As bacteriostaticum, 0.02% (W/v) sodium azide or 0.7% (w/v) chloroform can be used.

After about 48 hours at room temperature, the substrate was frozen and stored at $-25^{\circ}C$, or dryfrozen and stored at $+5^{\circ}C$.

Standard serum: Human serum with a known titer was inactivated ($56^{\circ}C$, 30 min.) prior to dilution by 1/25.

Dilution liquid: As a suitable dilution liquid can be used 10 mM tris-HCl buffer pH 7.6 with 0.1% (w/v) bovine serum albumine.

Method

(1)     As a reference solution for the photometer, 100 µl dilution liquid was applied to all wells in Column 1 of a microtitration plate of transparent plastics.

(2)     For checking (enzyme + substrate), i.e. reaction check, 25 µl dilution liquid, 25 µl DNase dilution and 50 µl DNA methyl green were applied to all wells in Column 2.

(3)     For substrate checking, 50 µl dilution liquid and 50 µl DNA methyl green were applied to all wells in Column 3.

(4)     24 µl dilution liquid was applied to all wells in Columns 4-12 with the exception of the wells in the first row.

For calculating the titers in standard sera and patient sera, Columns 4-12 on the first plate and Columns 2-12 on further plates were utilized. Column 1 was used throughout on all plates as a reference solution for the photometer. The description below relating to Columns 4-12 on the first plate also refers to Columns 2-12 on all successive plates.

(5)      50 µl inactivated (56°C, 30 min.) standard serum or patient serum, diluted to 1/25 with dilution liquid, was applied to the first row of wells in Columns 4-12. Three standard sera with various titers can suitably be applied to the wells in Columns 4-6.

(6)      From the first row of wells in Columns 4-12, dilution series were produced using the standard and patient sera from 1/25 (row A), 1/50 (row B) etc. up to 1/3200 (row H), with 25 µl in each well.

(7)      To Columns 4-12, 25 µl DNase dilution was added to each well. The plates were shaked on a special vibration table and were incubated in a 37°C thermostat for 15 minutes, whereafter the plates were vibrated once again after about half this period of time.

The total dilution of serum plus enzyme in the wells was 1/50 (row A), 1/100 (row B) etc. up to 1/6400 (row H).

(8)      To Columns 4-12 was added 50 µl DNA methyl green to each well. The plates were vibrated as above and incubated in a 37°C thermostat. After 15 minutes the plates were vibrated once again and were then left to remain in the thermostat for 19-24 hours.

Reading cycle: The reaction was read off with the aid of a photometer for microtitration plates, Flow Laboratories Multiskan, Multiskan MC, Dynatech MR 580-, Dynatech MR600-/ Microelisa Auto Reader or the like.

The reaction is optimally read at 640 nm but can also be read at 575-680 nm or 422 nm. Some of the photometer devices provide the possibility of correcting the absorbance values with regard to scratched and uneven plates, which can be done at 465-505 nm, or preferably, at 700 nm and a wider wavelength range.

A suitable minicomputer with flex disc unit and printer such as Commodore CBM Model 8032 or Luxor ABC 800 or the like can be connected to the photometer device.

A program has been developed which displays the data on a screen and allows for the operator to correct obviously erroneous values, whereafter the output is typed out on the printer in table form. All data are stored on a flex disc for further processing and filing. The program calculates the titers for patient and check sera according to the following definitions:

Positive reaction (+) is defined as the absorbance for the sample being greater than or equal to the average value of the absorbance for DNA methyl green minus 50% (Column 3).

Negative reaction (-) is defined as the absorbance for the sample being less than or equal to the average value of enzyme + DNA methyl green plus 50% (Column 2).

Positive/negative reaction (+/-) is defined as the absorbance for the sample lying between the above-mentioned values, and is considered as being a negative reaction.

The ADNase titer is stated as the reciprocal value of the highest total dilution of serum plus DNase B enzyme in the well (the method under Point 7) which was defined as a positive reaction according to the above.

A printout example of the output result from a determination of the titers for standard and patient sera according to the above is shown in the form of the following table.

Plate : 1

|   | 1 | 2 | 3 | 4 | 5 | 6 | 7 | 8 | 9 | 10 | 11 | 12 |
|---|---|---|---|---|---|---|---|---|---|---|---|---|
| A | 7.998 | 0.035 | 0.229 | 0.250 | 0.276 | 0.268 | 0.238 | 0.201 | 0.238 | 0.222 | 0.243 | 0.223 |
| B | 0.003 | 0.036 | 0.284 | 0.264 | 0.278 | 0.261 | 0.264 | 0.251 | 0.269 | 0.241 | 0.266 | 0.247 |
| C | 9.998 | 0.027 | 0.268 | 0.223 | 0.240 | 0.226 | 0.246 | 0.228 | 0.266 | 0.254 | 0.267 | 0.121 |
| D | 0.001 | 0.035 | 0.284 | 0.156 | 0.268 | 0.042 | 0.061 | 0.119 | 0.265 | 0.214 | 0.274 | 0.034 |
| E | 9.997 | 0.029 | 0.277 | 0.050 | 0.185 | 0.031 | 0.033 | 0.029 | 0.226 | 0.051 | 0.261 | 0.023 |
| F | 0.001 | 0.034 | 0.286 | 0.042 | 0.054 | 0.033 | 0.033 | 0.035 | 0.069 | 0.036 | 0.094 | 0.027 |
| G | 9.998 | 0.024 | 0.278 | 0.031 | 0.034 | 0.027 | 0.022 | 0.025 | 0.030 | 0.025 | 0.031 | 0.026 |
| H | 0.003 | 0.027 | 0.280 | 0.038 | 0.035 | 0.034 | 0.033 | 0.032 | 0.035 | 0.032 | 0.033 | 0.026 |

|   | 1 | 2 | 3 | 4. | 5 | 6 | 7 | 8 | 9 | 10 | 11 | 12 |
|---|---|---|---|---|---|---|---|---|---|---|---|---|
| A | – | – | + | + | + | + | + | + | + | + | + | + |
| B | – | – | + | + | + | + | + | + | + | + | + | + |
| C | – | – | + | + | + | + | + | + | + | + | + | +/– |
| D | – | – | + | + | + | – | +/– | +/– | + | + | + | – |
| E | – | – | + | +/– | + | – | – | – | + | +/– | + | – |
| F | – | – | + | – | +/– | – | – | – | +/– | – | +/– | – |
| G | – | – | + | – | – | – | – | – | – | – | – | – |
| H | – | – | + | – | – | – | – | – | – | – | – | – |

Titers (row): 2 :  < 50   3 : > 6400   4 :   400   5 :   800   6 : 200
7 :   200   8 :   200   9 :   800   10 :   400   11 :   800   12 : 100

Negative limit:     < .046
Positive limit:     > .136

0170760

CLAIMS

1. Method in serologic diagnostics of β-streptococcal infections by which streptococcal DNase B is neutralized with patient serum on microtitration plates, characterized in that automatic reading is performed in a photometer for microtitration plates.

2. Method as claimed in Claim 1, characterized in that DNA methyl green is used as a substrate, and in that the plate is illuminated with monochromatic light of 575-680 nm or 422 nm but preferably 640 nm.

3. Method as claimed in Claim 1, characterized in that DNA is used as a substrate, in that microtitration plates made of UV-transparent plastics are used, and in that said plates are illuminated with monochromatic UV-light of wavelength 260 or 254 nm.

4. Method as claimed in Claim 1, characterized in that DNA is used as a substrate, in that microtitration plates made of UV-transparent plastics are used, and in that said plates are illuminated with monochromatic light whereby the fluorescence is read off.

5. Method as claimed in any one of Claims 1 - 4, characterized in that readings are performed on a series of serum dilutions.

6. Method as claimed in any one of Claims 1 - 4, characterized in that the measurements are performed repeatedly on one and the same sample at specific time intervals.

7. Method as claimed in any one of the preceding claims, characterized in that the analysis results are automatically processed by connecting a minicomputer to the photometer device.

8. Utilization of a photometer device provided with a 640 nm interference filter at ADNase testings for reading off the patient serum inhibition of the decolorization reaction with streptococcal deoxyribonuclease B and DNA methyl green on microtitration plates.

9. Utilization of a photometer device provided with a 260 or a 254 nm filter at ADNase testings for reading off the patient serum inhibition of the reaction between streptococcal deoxyribonuclease B and DNA and patient serum on microtitration plates.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 69, no. 1, 1st July 1968, page 131, no. 1382n, Columbus, Ohio, US; J. NELSON et al.: "Streptococcal anti-deoxyribonuclease B: microtechnique determination" & J. LAB. CLIN. MED. 1968, 71(5), 867-873 (Cat. D) * Abstract * | 1-9 | G 01 N 33/569 G 01 N 33/573 C 12 Q 1/34 G 01 N 35/00 G 01 N 21/25 |
| Y | ANALYTICAL CHEMISTRY, vol. 56, no. 8, July 1984, pages 921A-931A, Easton, Pennsylvania, US; D. MONROE: "Immunoassay" * Pages 926A,928A * | 1,3,4, 7 | |
| Y | FR-A-2 425 073 (MILES LABORATORIES INC.) * Whole document * | 1,2,8 | |
| A | FR-A-2 242 685 (BEHRINGWERKE AG) * Pages 1-4; examples 4,5 * | 1,5 | **TECHNICAL FIELDS SEARCHED (Int. Cl.4)** G 01 N C 12 Q |
| A | EP-A-0 049 849 (BEHRINGWERKE AG) | | |
| A | GB-A-2 005 410 (LABORATOIRE DE RECHERCHE API) | | |

The present search report has been drawn up for all claims

| Place of search | Date of completion of the search | Examiner |
|---|---|---|
| THE HAGUE | 18-04-1985 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82